# FASCICULE DE BREVET EUROPEEN

(11) **EP 1 352 629 B1**
(45) Date de publication et mention de la délivrance du brevet: **22.06.2011**
(21) Numéro de dépôt: 03290848.5
(22) Date de dépôt: 04.04.2003
(51) Int. Cl.: A61Q 7/00, A61K 8/49

(54) **Utilisation d'un dérivé d'acide pyridine-dicarboxylique ou de l'un de ses sels pour stimuler ou induire la pousse des fibres kératiniques et/ou stopper leur chute**
Verwendung eines Pyridindicarbosäurederivates oder eines Salzes davon, zur Stimulation oder Anregung von keratinischen Faserwachstum und/oder zur Verhinderung von dessen Verlust
Use of a pyridine-dicarboxylic acid derivative or a salt thereof to stimulate or induce keratinic fiber growth and/or to prevent its loss

(30) Priorité: 11.04.2002 FR 0204527
(43) Date de publication de la demande: 15.10.2003
(73) Titulaire: L'Oréal, 75008 Paris (FR)
(72) Inventeur: Dalko, Maria, 91190 Gif S/Yvette (FR); Loussouarn, Geneviève, 92110 Clichy (FR); El Rawadi, Charles, 92500 Rueil-Malmaison (FR); Boulle, Christophe, 77400 Lagny S/Marne (FR); Bernard, Bruno, 92200 Neilly sur Seine (FR)
(74) Mandataire: Miszputen, Laurent

(56) Documents cités:
- FR-A- 2 677 247
- US-A- 5 472 687
- PATENT ABSTRACTS OF JAPAN vol. 014, no. 090 (C-0691), 20 février 1990 (1990-02-20) & JP 01 301612 A (BANYU PHARMACEUT CO LTD), 5 décembre 1989 (1989-12-05) & JP 01 301612 A (BANYU PHARMACEUT CO LTD) 5 décembre 1989 (1989-12-05)

## Description

L'invention a pour objet l'utilisation d'une quantité efficace d'un dérivé d'acide pyridine dicarboxylique ou de l'un de ses sels dans une composition destinée à induire et/ou stimuler la croissance des fibres kératiniques humaines et en particulier des cheveux et des cils humains et/ou freiner leur chute. Elle concerne en outre un procédé de traitement cosmétique destiné à stimuler la croissance des fibres kératiniques humaines telles que les cheveux et les cils et/ou freiner leur chute.

La croissance des cheveux et leur renouvellement sont principalement déterminés par l'activité des follicules pileux et leur environnement matriciel. Leur activité est cyclique et comporte essentiellement trois phases à savoir la phase anagène, la phase catagène et la phase télogène.
A la phase anagène (phase active ou de croissance), qui dure plusieurs années et au cours de laquelle les cheveux s'allongent, succède une phase catagène très courte et transitoire qui dure quelques semaines, puis une phase télogène ou phase de repos qui dure quelques mois.
A la fin de la période de repos, les cheveux tombent et un autre cycle recommence. La chevelure se renouvelle donc en permanence et sur les 150 000 cheveux environ que comporte une chevelure, 10% environ sont au repos et seront remplacés dans les mois qui viennent.

La chute ou perte naturelle des cheveux peut être estimée, en moyenne, à quelques cent cheveux par jour pour un état physiologique normal. Ce processus de renouvellement physique permanent subit une évolution naturelle au cours du vieillissement, les cheveux deviennent plus fins et leurs cycles plus courts.

En outre, différentes causes peuvent entraîner une perte importante, temporaire ou définitive, des cheveux. La chute des cheveux, en particulier l'alopécie, est essentiellement due à des perturbations du renouvellement capillaire. Ces perturbations entraînent dans un premier temps l'accélération de la fréquence des cycles au détriment de la qualité des cheveux, puis de leur quantité. Il se produit une miniaturisation progressive des bulbes, avec conjointement, un isolement de ceux-ci par épaississement progressif de la matrice collagénique péri-folliculaire ainsi que de la gaine conjonctive externe. La revascularisation autour du follicule pileux est donc rendue plus difficile cycle après cycle. Les cheveux régressent, se miniaturisant jusqu'à n'être plus qu'un duvet non pigmenté et ce phénomène entraîne un appauvrissement progressif de la chevelure.

Des zones sont touchées préférentiellement, notamment les golfes temporaux ou frontaux chez l'homme, et chez les femmes, on constate une alopécie diffuse du vertex.

Le terme alopécie recouvre aussi toute une famille d'atteintes du follicule pileux ayant pour conséquence finale la perte définitive, partielle ou générale des cheveux. Il s'agit plus particulièrement de l'alopécie androgénique. Dans un nombre important de cas, la chute précoce des cheveux survient chez des sujets prédisposés génétiquement, il s'agit alors d'alopécie andro-chrono-génétique ; cette forme d'alopécie concerne notamment les hommes.

Il est connu, par ailleurs, que certains facteurs tels qu'un déséquilibre hormonal, un stress physiologique, la malnutrition, peuvent accentuer le phénomène. En outre, la chute ou l'altération des cheveux peut être en relation avec des phénomènes saisonniers.

De façon générale, tout facteur influençant ces processus, à savoir l'accélération de la fréquence des cycles, la miniaturisation progressive des bulbes, l'épaississement progressif de la matrice collagénique péri-folliculaire, l'épaississement de la gaine conjonctive externe, et la diminution de la vascularisation, aura un effet sur la croissance des follicules pileux.

FR 2 677 247 et JP 1-301 612 décrivent l'utilisation de pyridine N-oxyde pour freiner la chute des cheveux et pour induire et stimuler leur croissance.

La demanderesse a trouvé que des acides pyridine-dicarboxyliques et certains dérivés de ces acides pyridine-dicarboxyliques, notamment des esters et des amides, sont d'une façon surprenante dotés d'une activité favorable pour maintenir et/ou améliorer la densité des fibres kératiniques humaines et en particulier la densité capillaire chez l'être humain et/ou réduire l'hétérogénéité des diamètres des fibres kératiniques et plus spécialement des cheveux chez l'être humain. Par augmenter la densité des fibres kératiniques, notamment capillaire, on entend augmenter le nombre des fibres kératiniques, notamment des cheveux par cm² de peau et notamment de cuir chevelu.

La présente invention a donc pour objet l'utilisation d'au moins un dérivé d'acide pyridine-dicarboxylique de formule générale (I) ou de l'un de ses sels 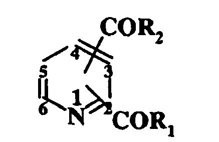 dans laquelle R₁ et R₂ représentent indépendamment l'un de l'autre OH, OR', -NH₂, -NHR', -NR'R", et
R' et R" représentent indépendamment l'un de l'autre, un radical alkyle en C₁-C₁₈, linéaire ou ramifié, saturé ou insaturé, ou un radical aryle, ce radical aryle ou alkyle étant éventuellement substitué par au moins un groupe OH, alkoxy, acyloxy, amino, ou alkylamino, ou R' et R" représentent ensemble un hétérocycle,
dans une composition cosmétique de soin et/ou de maquillage des fibres kératiniques ou pour la préparation d'une composition de soin et/ou de traitement des fibres kératiniques, pour ou destinée à induire et/ou stimuler la croissance des fibres kératiniques humaines et/ou freiner leur chute et/ou augmenter leur densité.

L'invention a encore pour objet l'utilisation cosmétique d'au moins un dérivé d'acide pyridine dicarboxylique de formule (I) ou l'un de ses sels, tel que défini ci-dessus dans une composition cosmétique, comme agent pour induire et/ou stimuler la croissance des fibres kératiniques humaines et/ou freiner leur chute et/ou augmenter leur densité.

Les fibres kératiniques humaines auxquelles s'applique l'invention sont notamment les cheveux, les sourcils, les cils, les poils de barbe, de moustache et les poils pubiens. Plus spécialement, l'invention s'applique aux cheveux et/ou aux cils humains.

Aussi, l'invention se rapporte encore à l'utilisation cosmétique d'au moins un dérivé d'acide pyridine dicarboxylique de formule (I) ou de l'un de ses sels, dans une composition cosmétique de soin capillaire d'être humain, pour traiter l'alopécie androgénique, ainsi qu'à l'utilisation d'au moins un dérivé d'acide pyridine dicarboxylique de formule (I) ou de l'un de ses sels pour la préparation d'une composition de soin capillaire d'être humain, destinée à traiter l'alopécie androgénique. Ainsi, cette composition permet de maintenir en bon état la chevelure et/ou lutter contre la chute naturelle des cheveux des hommes.

L'invention a encore pour objet l'utilisation cosmétique d'au moins un dérivé d'acide pyridine dicarboxylique de formule (I) ou de l'un de ses sels, tel que défini ci-dessus, dans une composition cosmétique de soin et/ou de maquillage des cils d'être humain ou pour la préparation d'une composition de soin et/ou de traitement des cils d'être humain, pour ou destinée à induire et/ou stimuler la croissance des cils et/ou augmenter leur densité. Cette composition permet ainsi de maintenir en bon état les cils et/ou améliorer leur état et/ou leur aspect.

Selon l'invention, "au moins un" dérivé signifie un ou plusieurs (2, 3 ou plus) dérivés.

Les dérivés auxquels s'applique l'invention sont connus en tant que tels ; ils peuvent être fabriqués de façon connue.

Ainsi, les acides pyridine-2,4 et 2,5-dicarboxyliques ont été décrits comme de très faibles inhibiteurs de la proline hydroxylase (K. Majamaa et al., Eur. J. Biochem. 138, 1984, 239-245). Sharir et al. ont suggéré d'utiliser l'acide pyridine-2,4-dicarboxylique pour retarder la cicatrisation d'une plaie chirurgicale (Current Eye Research, vol. 12(6), 1993, 553-559).

Le brevet US 4 717 727 enseigne que les esters d'acides pyridine-2,4 et 2,5-dicarboxyliques peuvent être utilisés dans le traitement des pathologies liées au métabolisme du collagène, d'une part, et comme immunosuppresseur, d'autre part.

A la connaissance de la demanderesse, aucun document de l'art antérieur n'enseigne ni ne suggère que les dérivés de l'acide pyridine-dicarboxylique ou un de leurs sels, visés par la présente invention, ont la propriété d'induire et/ou de stimuler la croissance des fibres kératiniques humaines et en particulier des cheveux humains et des cils et/ou de freiner leur chute et/ou d'augmenter leur densité.

Le radical alkyle en C₁-C₁₈ est de préférence un alkyle saturé ou insaturé comprenant de 1 à 10 atomes de carbone, tel que méthyle, éthyle, tertio-butyle, iso-propyle, hexyle et autres. L'alkyle peut contenir au moins une double liaison carbone-carbone ou une triple liaison carbone-carbone, comme par exemple -CH=CH₂, -CH₂-CH=CH-CH₃, -CH₂-C ≡CH.

Selon la présente invention, on entend par « alkoxy », un groupement -O-R dans lequel R est un groupement alkyle en C₁-C₁₈ comme défini précédemment. On entend par « acyloxy » un groupement -O-CO-R, dans lequel R est un groupement alkyle en C₁-C₁₈ comme défini précédemment. On entend par « alkylamino », un groupement -NH-R, dans lequel R est un groupement alkyle en C₁-C₁₈ comme défini précédemment.

Le radical aryle peut représenter le radical phényle ou naphtyle.

Lorsque R' et R" représentent ensemble un hétérocycle, ils peuvent représenter un cycle de 4 à 7 atomes et mieux de 5 à 6 atomes, comportant de 1 à 4 hétéroatomes choisis parmi O, S, N, ce cycle pouvant être saturé ou non. Comme hétérocycle, on peut citer les cycles pipéridine, morpholine, imidazole, pyrazole, pipérazine, pyrrolidine, thiazolidine.

En particulier, R' et R" représentent un radical alkyle en C₁-C₁₈ et mieux en C₁-C₁₀ éventuellement substitué par un groupe alkoxy ou acyloxy.

R₁ et R₂ représentent de préférence indépendamment l'un de l'autre OH, OCH₃, OCH₂ - CH₃, 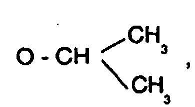 O-CH₂-O-COCH₃ , NHCH₂-CH₂ -CH₃ , NH-CH₂-CH₂OH, NH-CH₂-CH₂-CH₂OH, NH-CH₂-CH₂OCH₃.

COR₁ et COR₂ sont de préférence respectivement en position 2 et 3, ou 2 et 4 du noyau pyridine. Ils peuvent toutefois être respectivement en position 2 et 5.

Selon un mode préféré de réalisation de l'invention, on utilise les dérivés d'acide pyridine-dicarboxylique suivants :
- l'acide pyridine-2,4-dicarboxylique ou son sel de zinc ou de sodium,
- l'acide pyridine-2,3-dicarboxylique ou son sel de zinc ou de sodium,
- le pyridine-2,4-dicarboxylate de diméthyle,
- le pyridine-2,3-dicarboxylate de diméthyle,
- le pyridine-2,4-dicarboxylate de diéthyle,
- le pyridine-2,3-dicarboxylate de diéthyle,
- le pyridine-2,5-dicarboxylate de diéthyle,
- le pyridine-2,5-dicarboxylate de diméthyle,
- le pyridine-2,4-dicarboxylate de di-isopropyle,
- la di(n-propylamido)-2,4-pyridine, (dérivé de formule (I) avec R₁ et R₂ représentent NH-(CH₂)₂-CH₃,
- le pyridine-2,4-dicarboxylate de di(acétyloxyméthyle) (dérivé de formule (I) tel que R₁ et R₂ représentent O-CH₂-O-COCH₃),
- la di(2-hydroxy éthylamido-2,4 pyridine),
- la di(3-hydroxy propylamido-2,4 pyridine).

On utilise avantageusement les esters de l'acide pyridine-dicarboxylique, car ils sont dotés d'une meilleure pénétration cutanée, et en particulier le pyridine -2,4 dicarboxylate de diéthyle.

Par sels de composé de formule (I) on entend selon l'invention les sels organiques ou inorganiques d'un composé de formule (I), ces sels étant physiologiquement acceptables.

Comme sels inorganiques utilisables selon invention on peut citer les sels doubles de sodium ou de potassium ainsi que les sels de zinc (Zn²⁺), de calcium (Ca²⁺), de cuivre (Cu²⁺), de fer (Fe²⁺), de strontium (Sr²⁺), de magnésium (Mg²⁺), de manganèse (Mn²⁺) ; les hydroxydes, les carbonates et les chlorures.

Les sels organiques utilisables selon l'invention sont par exemple les sels tri-éthanolamine, mono-éthanolamine, di-éthanolamine, hexadécylamine, N,N,N',N'-tétrakis-(hydroxy-propyl-2) éthylène di-amine et tris-hydroxyméthyl aminométhane.

On comprend ainsi que dans le texte, sous réserve d'indication contraire, l'emploi du terme composé de formule (I) doit être compris comme signifiant le composé de formule (I) sous forme salifiée ou non.

La quantité efficace d'un composé de formule (I) ou de l'un de ses sels correspond bien entendu à la quantité nécessaire pour obtenir le résultat désiré (à savoir augmenter la densité des fibres kératiniques et notamment les cheveux ou favoriser leur pousse). L'homme du métier est donc en mesure d'évaluer cette quantité efficace qui dépend de la nature du composé utilisé et de la personne à laquelle on l'applique.

Pour donner un ordre de grandeur, selon l'invention, le composé de formule (I) ou l'un de ses sels peut être utilisé en une quantité représentant de 10⁻³ % à 10% du poids total de la composition et préférentiellement en une quantité représentant de 10⁻² % à 5% du poids total de la composition, par exemple de 0,5 % à 2 %.

Aussi, la composition doit contenir un milieu physiologiquement acceptable non toxique et susceptible d'être appliqué sur la peau, y compris le cuir chevelu et les paupières, ou sur les fibres kératiniques d'êtres humains. Par "cosmétique", on entend au sens de l'invention une composition d'aspect, d'odeur et de toucher agréables.

La composition selon l'invention peut être à usage cosmétique ou pharmaceutique. Préférentiellement, la composition selon l'invention est à usage cosmétique.

Le composé de formule (I) ou l'un de ses sels peut être utilisé dans une composition qui doit être ingérée, injectée ou appliquée sur la peau ou les fibres kératiniques (sur toute zone cutanée du corps et des fibres à traiter).

Selon l'invention, le composé de formule (I) ou l'un de ses sels peut être utilisé par la voie orale en une quantité de 0,1 à 300 mg par jour, par exemple de 5 à 10 mg/j.

Une composition préférée de l'invention est une composition à usage cosmétique d'application topique sur la peau et les fibres kératiniques, et plus spécialement sur le cuir chevelu, les cheveux et les cils.

Selon le mode d'application, cette composition peut se présenter sous toutes les formes galéniques normalement utilisées dans les domaines cosmétique et pharmaceutique.

Pour une application topique sur la peau, y compris le cuir chevelu, la composition peut avoir la forme notamment d'une solution ou suspension aqueuse, alcoolique ou hydro-alcoolique, d'une suspension ou d'une solution huileuse, d'une émulsion ou dispersion de consistance liquide ou semi-liquide obtenue par dispersion d'une phase grasse dans une phase aqueuse (H/E) ou inversement (E/H), d'une dispersion ou émulsion de consistance molle, d'un gel aqueux ou hydro-alcoolique ou huileux (anhydre), d'une poudre libre ou compactée à utiliser telle quelle ou à incorporer dans un milieu physiologiquement acceptable (excipient), ou encore de microcapsules ou microparticules, ou de dispersions vésiculaires de type ionique et/ou non ionique.

On peut également envisager une composition sous la forme d'une mousse ou encore sous forme de composition pour aérosol comprenant également un agent propulseur sous pression.

En particulier, la composition à application sur le cuir chevelu ou les cheveux peut se présenter sous forme d'une lotion de soin capillaire, par exemple d'application journalière ou bi-hebdomadaire, d'un shampooing ou d'un après-shampooing capillaire, en particulier d'application bi-hebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.

Par ailleurs, pour une application sur les cils ou les poils, la composition à laquelle s'applique invention peut se présenter sous forme d'un mascara, pigmenté ou non, à appliquer à la brosse sur les cils ou encore sur les poils de barbe ou de moustache.

Pour un emploi par injection, la composition peut se présenter sous forme de lotion aqueuse, de suspension huileuse par exemple sous forme de sérum. Pour un emploi par ingestion ou voie orale, elle peut se présenter sous forme de capsules, de granulés, de sirops buvables ou de comprimés.

Selon un mode de réalisation particulier, la composition à laquelle s'applique l'invention se présente sous forme de crème ou lotion capillaire, de shampooing, d'après-shampooing capillaire, de mascara capillaire ou pour cils.

Les quantités des différents constituants de la composition selon l'invention sont celles classiquement utilisées dans les domaines considérés. En outre, cette composition est préparée selon les méthodes usuelles. Elle peut ainsi se présenter sous forme d'une lotion, sérum, lait, crème H/E ou E/H, gel, onguent, pommade, poudre, baume, patch, tampon imbibé, savon, pain, mousse.

Lorsque la composition est une émulsion, la proportion de la phase grasse peut aller de 2 % à 80 % en poids, et de préférence de 5 % à 50 % en poids par rapport au poids total de la composition.

La phase aqueuse est ajustée en fonction de la teneur en phase grasse et en composé(s) (I) ainsi que de celle des éventuels ingrédients additionnels, pour obtenir 100% en poids. En pratique la phase aqueuse représente de 5 % à 99,9% en poids.

La phase grasse peut contenir des composés gras ou huileux, liquides à température ambiante (25°C) et pression atmosphérique (760 mm de Hg), généralement appelés huiles. Ces huiles peuvent être compatibles ou non entre elles et former une phase grasse liquide macroscopiquement homogène ou un système bi- ou triphasique.

La phase grasse peut, en plus des huiles, contenir des cires, des gommes, des polymères lipophiles, des produits "pâteux" ou visqueux contenant des parties solides et des parties liquides.

La phase aqueuse contient de l'eau et éventuellement un ingrédient miscible en toute proportion à l'eau comme les alcools inférieurs en C₁ à C₈ tel que l'éthanol, l'isopropanol, les polyols comme le propylène glycol, le glycérol, le sorbitol ou encore l'acétone ou l'éther.

Les émulsionnants et les coémulsionnants utilisés dans la composition sous forme d'émulsion sont choisis parmi ceux classiquement utilisés dans le domaine cosmétique ou pharmaceutique. L'émulsionnant et le coémulsionnant sont présents, dans la composition, en une proportion allant de 0,1 % à 30 % en poids, et de préférence de 0,5 % à 20 % en poids par rapport au poids total de la composition et mieux de 1 % à 8 %. Leur nature est, en outre, fonction du sens de l'émulsion. L'émulsion peut, en outre, contenir des vésicules lipidiques et notamment des liposomes.

Lorsque la composition est une solution ou un gel huileux, la phase grasse peut représenter plus de 90 % du poids total de la composition.

Avantageusement pour une application capillaire, la composition est une solution ou suspension aqueuse, alcoolique ou hydro-alcoolique et mieux une solution ou suspension eau/éthanol. La fraction alcoolique peut représenter de 5% à 99,9% et mieux de 8% à 80%.

Pour une application mascara, la composition est une dispersion de cire-dans-eau ou de cire dans huile, une huile gélifiée, un gel aqueux, pigmenté ou non.

La composition à laquelle s'applique l'invention peut contenir également des adjuvants habituels dans le domaine cosmétique ou pharmaceutique, choisis parmi les gélifiants ou épaississants hydrophiles ou lipophiles, les additifs hydrophiles ou lipophiles, les conservateurs, les antioxydants (caroténoïdes), les solvants, les parfums, les charges, les absorbeurs d'odeurs, les électrolytes, les neutralisants, les agents bloqueurs d'U.V. comme les filtres solaires, les polymères filmogènes, les actifs cosmétiques et pharmaceutiques à action bénéfique pour la peau ou les fibres kératiniques (comme les vitamines) et les matières colorantes, solubles ou non dans le milieu. Les quantités de ces différents adjuvants sont celles classiquement utilisées dans le domaine cosmétique, et par exemple de 0,01 % à 20 %, et mieux de 0,1 % à 10 % du poids total de la composition. Ces adjuvants, selon leur nature, peuvent être introduits dans la phase grasse, dans la phase aqueuse et/ou dans les vésicules lipidiques et notamment des liposomes.

Comme huiles ou cires utilisables dans l'invention, on peut citer les huiles minérales (huile de vaseline, isoparaffine hydrogénée), les huiles végétales (fraction liquide du beurre de karité, huile de tournesol, de soja, de germes de blé), les huiles animales (perhydrosqualène), les huiles de synthèse (huile de Purcellin, esters d'acide gras), les huiles ou cires siliconées (polydiméthylsiloxanes linéaires ou cycliques -cyclométhicone- phényltriméthicone), les huiles fluorées (perfluoropolyéthers), les cires d'abeille, de candellila, de riz, de carnauba, de paraffine ou de polyéthylène. On peut ajouter à ces huiles et cires des alcools gras et des acides gras (acide stéarique, linoléique, linolénique).

Comme émulsionnants utilisables dans l'invention, on peut citer par exemple le stéarate ou laurate de glycérol, le stéarate ou oléate de sorbitol polyoxyéthyléné (par exemple le polysorbate 60 et le mélange de PEG-6/PEG-32/Glycol Stéarate vendu sous la dénomination de Tefose^{®} 63 par la société Gattefosse), les (alkyl)diméthicones copolyol.

Comme solvants utilisables dans l'invention, on peut citer les alcools inférieurs, notamment l'éthanol et l'isopropanol, le propylène glycol, certaines huiles cosmétiques légères.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les Bentones, les sels métalliques d'acides gras comme les stéarates d'aluminium, la silice traitée hydrophobe, l'éthylcellulose et leurs mélanges.

La composition peut contenir d'autres actifs que ceux de formule (I) qui peuvent être hydrophiles, comme les protéines ou les hydrolysats de protéine, les acides aminés, les polyols, l'urée, l'allantoïne, les sucres et les dérivés de sucre, les vitamines hydrosolubles, les extraits végétaux (d'lridacées ou de soja), ces extraits pouvant alors contenir ou non des isoflavones ; ou lipophiles, comme le rétinol (vitamine A) et ses dérivés notamment ester (palmitate), le tocophérol (vitamine E) et ses dérivés, notamment ester (acétate palmitate), les acides gras essentiels comme les acides eicosatétraénoïque et eicosatriénoïque ou leurs esters et amides, les céramides, les huiles essentielles, les esters des hydroxy-acides, les phospholipides comme la lécithine ; ou solubles dans des solvants alcooliques comme les lactones (kawaïne) ; leurs mélanges.

Selon l'invention, on peut, entre autre, associer le composé de formule (I) ou l'un de ses sels à d'autres composés actifs additionnels favorisant en particulier la repousse des fibres kératiniques humaines et/ou limitant leur chute. Ces composés additionnels sont notamment choisis parmi :
- les hormones en particulier d'origine végétale par exemple l'estriol ou ses analogues, la thyroxine et ses sels, la progestérone ;
- les agents antibactériens tels que les macrolides, les pyranosides et les tétracyclines, et notamment l'érythromycine ;
- les agents antagonistes de calcium, comme la cinnarizine, le diltiazem, la nimodipine, le vérapamil et la nifédipine ;
- les extraits de micro-organismes notamment les extraits bactériens ;
- les agents modulant la différenciation et/ou la prolifération cutanée tels que l'acide rétinoïque et ses isomères, le rétinol et ses esters, la vitamine D et ses dérivés, les oestrogènes tels que l'oestradiol ;
- les agents modulant l'adhésion bactérienne sur la peau et /ou les muqueuses tels que le miel, notamment le miel d'acacias et certains dérivés de sucres ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les esters d'acide nicotinique dont notamment le nicotinate de tocophérol, le nicotinate de benzyle et les nicotinates d'alkyles en C₁-C₆ comme les nicotinates de méthyle ou d'hexyle ; les composés polyènes, tels que l'amphotéricine B, les composés de la famille des allylamines, tels que la terbinafine, ou encore l'octopirox, les dérivés du sélénium ou l'anthraline ;
- les agents antiviraux tels que l'acyclovir
- les agents anti-inflammatoires stéroïdiens tels que les corticostéroïdiens comme l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique, l'α-bisabolol ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxycarboxyliques ou bêta-cétocarboxyliques, leurs sels, amides ou esters, les lactones et leurs sels correspondants, et plus particulièrement les hydroxy-acides tels que l'acide glycolique, l'acide lactique, l'acide salicylique, l'acide citrique et de manière générale les acides de fruits, les dérivés de l'acide salicylique tels que ceux porteurs d'un radical alcanoyle ayant de 2 à 12 atomes de carbone en position 5 du cycle benzénique comme l'acide n-octanoyl-5-salicylique ;
- les agents anti-radicaux libres tels que l'alpha-tocophérol ou ses esters, les superoxyde dismutases, certains chélatants de métaux ou l'acide ascorbique et ses esters ;
- les anti-séborrhéiques tels que la progestérone ;
- les antipelliculaires comme l'octopirox ou la pyrithione de zinc ;
- les antiacnéiques comme l'acide rétinoïque ou le peroxyde de benzoyle ;
- les vasodilatateurs comme les dérivés de pyrimidine, comme le 2,4-diamino 6-pipéridinopyrimidine 3-oxyde ou "minoxidil" décrit dans les brevets US 4 139 619 et US 4 596 812, le diazoxide ;
- les agents diminuant la chute des cheveux comme l'aminexil, le 6-O-[(9Z,12Z)-octadéca-9,12-diènoyl]hexapyranose ;
- les agents antiandrogènes tels que l'oxendolone, la spironolactone, le diéthylstilbestrol et la flutamide ;
- les inhibiteurs stéroïdiens ou non stéroïdiens des 5-α-réductases tels que le finastéride ;
- les agonistes potassiques tels que la cromakalim et le nicorandil ;
- les agonistes du récepteur FP (récepteur aux prostaglandines de type F) tels que le latanoprost, le bimatoprost, le travoprost, l'unoprostone ;
- leurs mélanges

La composition selon l'invention peut, en outre, comprendre des substances telles que les antagonistes de substance P, de CGRP ou de bradykinine et les inhibiteurs de NO synthase, composés décrits comme étant actifs dans le traitement des peaux sensibles et comme présentant des effets anti-irritants, en particulier vis-à-vis de composés irritants éventuellement présents dans les compositions.

Avantageusement, la composition selon l'invention contient au moins un actif additionnel favorisant la repousse des fibres kératiniques humaines et/ou limitant la chute choisi parmi l'aminexil, les agonistes du récepteur FP et les vasodilatateurs et plus spécialement choisi parmi l'aminexil, le minoxidil, le latanoprost et le travoprost.

On peut également envisager que la composition comprenant au moins le composé de formule (I) ou l'un de ses sels soit sous forme liposomée, telle que notamment décrite dans la demande de brevet WO 94/22468 déposée le 13 octobre 1994 par la société Anti Cancer Inc. Ainsi, le composé encapsulé dans les liposomes peut être délivré sélectivement au niveau du follicule pileux.

La composition selon l'invention en particulier cosmétique peut être appliquée sur les zones alopéciques du cuir chevelu et des cheveux d'un individu, et éventuellement laissée en contact plusieurs heures et éventuellement rincée.

On peut, par exemple, appliquer la composition contenant une quantité efficace d'un composé de formule (I) ou de l'un de ses sels, le soir, garder celle-ci en contact toute la nuit et éventuellement effectuer un shampooing le matin. Ces applications peuvent être renouvelées quotidiennement pendant un ou plusieurs mois suivant les individus.

Ainsi, la présente invention a également pour objet un procédé de traitement cosmétique des fibres kératiniques humaines et/ou de la peau d'où émergent lesdites fibres, y compris du cuir chevelu, caractérisé par le fait qu'il consiste à appliquer sur les fibres kératiniques et/ou la peau d'où émergent lesdites fibres, une composition cosmétique comprenant au moins un dérivé de formule (I) ou l'un de ses sels, à laisser celle-ci en contact avec les fibres kératiniques et/ou la peau d'où émergent lesdites fibres, et éventuellement à rincer les fibres et/ou la peau.

Ce procédé de traitement présente bien les caractéristiques d'un procédé cosmétique dans la mesure où il permet d'améliorer l'esthétique des fibres kératiniques humaines en leur donnant une plus grande vigueur et un aspect amélioré. En outre, il peut être utilisé quotidiennement pendant plusieurs mois, sans prescription médicale.

Plus spécialement, la présente invention a pour objet un procédé de soin cosmétique des cheveux et/ou du cuir chevelu humains, en vue d'améliorer leur état et/ou leur aspect, caractérisé en ce qu'il consiste à appliquer sur les cheveux et/ou le cuir chevelu, une composition cosmétique comprenant au moins un dérivé de formule (I) ou l'un de ses sels, à laisser celle-ci au contact des cheveux et/ou du cuir chevelu, et éventuellement à rincer les cheveux et/ou le cuir chevelu.

L'invention a encore pour objet un procédé de soin cosmétique et/ou de maquillage des cils humains, en vue d'améliorer leur état et/ou leur aspect, caractérisé en ce qu'il consiste à appliquer une composition de mascara comprenant au moins un composé de formule (I) ou l'une de ses sels et à laisser celle-ci au contact des cils. Cette composition de mascara peut être appliquée seule ou en sous-couche d'un mascara pigmenté classique et être éliminée comme un mascara pigmenté classique.

L'invention a encore pour objet une composition de soin ou de maquillage des fibres kératiniques, comprenant dans un milieu physiologiquement acceptable, en particulier cosmétique, au moins un dérivé de formule (I) ou l'une de ses sels, tel que défini précédemment, et au moins un actif additionnel favorisant la repousse des fibres kératiniques humaines et/ou limitant la chute choisi parmi l'aminexil, les agonistes du récepteur FP et les vasodilatateurs et plus spécialement choisi parmi l'aminexil, le minoxidil, le latanoprost et le travoprost.

On va maintenant donner à titre d'illustration des exemples qui ne sauraient limiter en aucune façon la portée de l'invention.

Ces compositions sont obtenues par les techniques habituelles couramment utilisées en cosmétique ou en pharmacie.

### EXEMPLE 1 : Lotion capillaire

- Pyridine-2,4-dicarboxylate de diéthyle 0,80 g
- Propylène glycol 10,00 g
- Alcool isopropylique qsp 100,00 g

On applique 1 ml de cette lotion sur le cuir chevelu, à la fréquence de une à deux fois par jour, en massant légèrement le cuir chevelu pour faire pénétrer l'actif dans l'épiderme. La chevelure est ensuite séchée à l'air libre. Cette lotion assure une diminution de la chute des cheveux, et améliore l'aspect et l'état des cheveux.

### EXEMPLE 2 : Lotion capillaire

- Pyridine-2,4-dicarboxylate de diéthyle 2 g
- Propylène glycol 30,00 g
- Alcool éthylique 40,00 g
- Eau qsp 100,00 g

On applique cette lotion sur le cuir chevelu, une à deux fois par jour, à raison d'1 ml par application, en massant légèrement le cuir chevelu. La chevelure est ensuite séchée à l'air libre.

### EXEMPLE 3 : Lotion capillaire

- Pyridine-2,4-dicarboxylate de diéthyle 2 g
- Alcool éthylique 40,00 g
- Eau qsp 100,00 g

On applique cette lotion sur le cuir chevelu, une à deux fois par jour, à raison d'1 ml par application, en massant légèrement le cuir chevelu. On sèche ensuite les cheveux à l'air libre ou à l'aide d'un sèche cheveux.

### EXEMPLE 4 : Lotion capillaire

- Pyridine-2,4-dicarboxylate de di-isopropyle 2 g
- Propylène glycol 30,00 g
- Alcool éthylique 40,00 g
- Eau qsp 100,00 g

On applique 1 ml de cette lotion sur le cuir chevelu, à la fréquence de une à deux fois par jour, en massant légèrement le cuir chevelu. On sèche ensuite les cheveux à l'air libre ou à l'aide d'un sèche cheveux.

### EXEMPLE 5 : Lotion capillaire épaissie

- Pyridine-2,4-dicarboxylate de diméthyle 0,50 g
- Kawaïne 2,00 g
- Klucel G^{®}* 3,50 g
- Alcool éthylique qsp 100,00 g

On applique cette lotion épaissie sur le cuir chevelu, une à deux fois par jour, à raison d'1 ml par application, en massant légèrement le cuir chevelu. On sèche ensuite les cheveux à l'air libre ou à l'aide d'un sèche cheveux.

### EXEMPLE 6 : Lotion capillaire

- Pyridine-2,4-dicarboxylate de diméthyle 1,00 g
- Dowanol PM^{®}** 20,00 g
- Klucel G^{®}* 3,00 g
- Alcool éthylique 40,00 g
- Eau qsp 100,00 g

On applique cette lotion épaissie sur le cuir chevelu, une à deux fois par jour, à raison d'1 ml par application, en massant légèrement le cuir chevelu. On sèche ensuite les cheveux à l'air libre ou à l'aide d'un sèche cheveux.

### EXEMPLE 7 : Lotion capillaire

- Sel de sodium d'acide pyridine-2,4-dicarboxylique 5,00 g
- Alcool éthylique 20,00 g
- Eau qsp 100,00 g

On applique cette lotion sur le cuir chevelu, une à deux fois par jour, à raison d'1 ml par application.

### EXEMPLE 8 : Lotion capillaire

- Pyridine-2,4-dicarboxylate de diéthyle 2 g
- Alcool éthylique 50,00 g
- Aminexil 1,50 g
- Eau qsp 100,00 g

On applique cette lotion sur le cuir chevelu, une à deux fois par jour, à raison d'1 ml par application, en massant légèrement le cuir chevelu. On sèche ensuite les cheveux à l'air libre ou à l'aide d'un sèche cheveux.

### EXEMPLE 9 : Lotion capillaire

- Pyridine-2,4-dicarboxylate de diéthyle 2 g
- Aminexil 1,5 g
- Propylène glycol 30,00 g
- Alcool éthylique 40,00 g
- Eau qsp 100,00 g

On applique cette lotion sur le cuir chevelu, une à deux fois par jour, à raison d'1 ml par application, en massant légèrement le cuir chevelu. La chevelure est ensuite séchée à l'air libre.

### EXEMPLE 10 : Composition pour voie orale

On prépare de manière classique pour l'homme du métier, des capsules molles ayant la composition suivante:
- huile de soja hydrogénée 40 mg
- huile de germes de blé 95 mg
- lécithine de soja 20 mg
- tocophérols naturels 5 mg
- acide ascorbique 30 mg
- Pyridine-2,4-dicarboxylate de diméthyle 10 mg

On utilise cette composition une à deux fois par jour, à raison d'une capsule par absorption.
* : Hydroxypropylcellulose vendue par la société Hercules
** : Monométhyléther de propylèneglycol vendu par la société Dow Chemical

### EXEMPLE 11 : Lotion capillaire

- Sel de Na de l'acide pyridine 2,4 dicarboxylique 2 g
- Aminexil 1,5 g
- Alcool éthylique 40 g
- Eau qsp 100,00 g

On applique cette lotion sur le cuir chevelu, une à deux fois par jour, à raison d'1 ml par application, en massant légèrement le cuir chevelu. La chevelure est ensuite séchée à l'air libre.

### EXEMPLE 12 : Lotion capillaire

- Pyridine 2,4 dicarboxylate de diéthyle 2 g
- Latanoprost 0,005g
- Propylène glycol 3 0 g
- Alcool éthylique 40 g
- Eau qsp 100,00 g

On applique cette lotion sur le cuir chevelu, une à deux fois par jour, à raison d'1 ml par application, en massant légèrement le cuir chevelu. La chevelure est ensuite séchée à l'air libre.

### EXEMPLE 13 : Activité in vivo

### 1^{ère} étude :

L'acide pyridine-2,4-dicarboxylique a été testé *in vivo* chez 25 hommes présentant une alopécie androgénogénétique de stade III à V selon la classification d'Hamilton.
Il a été étudié à la concentration de 2%, formulé dans une lotion ternaire à base de propylène glycol, éthanol et eau.
Chaque volontaire a été traité une fois par jour, 5 jours par semaine pendant 3 mois, une zone du cuir chevelu recevant 30µl de la lotion contenant l'actif et une autre zone recevant le véhicule seul (placebo).
L'évaluation de l'efficacité des traitements s'est faite par détermination de la densité pilaire totale de cheveux par cm² (DE) au moyen de la technique du phototrichogramme. Cet examen a été réalisé avant traitement (M0) et après 3 mois de traitement (M3).

Les résultats (moyenne ± écart type) et les variations par rapport à M0 sont présentés dans le tableau suivant :

| | Placebo | | acide pyridine-2,4-dicarboxylique à 2% | |
|---|---|---|---|---|
| | Nb cheveux / par cm² (DE) | Variation DE (%) | Nb cheveux / par cm² (DE) | Variation DE (%) |
| M0 | 217± 50 | / | 213± 45 | / |
| M3 | 218 ± 57 | 0% | 224 ± 53 | 4,4% |

Les résultats montrent une augmentation de la densité totale pour les zones traitées avec l'acide pyridine-2,4-dicarboxylique alors que celles ayant reçu le placebo n'évoluent pas.

### 2^{éme} étude :

L'acide pyridine-2,4-dicarboxylique a été testé sous forme de sel double de Na formulé à la concentration de 5% dans une lotion hydro-alcoolique.
L'étude a été conduite versus placebo chez 22 hommes présentant une alopécie androgénogénétique de stade III à V selon la classification d'Hamilton.
Chaque volontaire a été traité une fois par jour, 5 jours par semaine pendant 3 mois, une zone du cuir chevelu recevant 30 µl de la lotion contenant l'actif et une autre zone recevant le véhicule seul.
L'évaluation de l'efficacité des traitements s'est faite par détermination de la densité pilaire totale de cheveux par cm² (DE) au moyen de la technique du phototrichogramme. Cet examen a été réalisé avant traitement (M0) et après 3 mois de traitement (M3).
Les résultats (moyenne ± écart type) et la moyenne de la variation par rapport à M0 sont présentés dans le tableau suivant :

| | Placebo | | di-sel de Na d'acide pyridine-2,4-dicarboxylique à 5% | |
|---|---|---|---|---|
| | Nb cheveux / par cm² (DE) | Variation DE (%) | Nb cheveux / par cm² (DE) | Variation DE (%) |
| M0 | 247± 52 | / | 249 ± 50 | / |
| M3 | 236 ± 52 | -4.6% | 246 ± 50 | -0,97% |

Les résultats montrent une diminution de la densité totale sur les zones ayant reçu le placebo. Cette perte de cheveux est 4 fois moins importante pour les zones ayant reçu le sel double de Na de l'acide pyridine-2,4-dicarboxylique.

Les deux études menées *in vivo* sur des sujets alopéciques montrent comparativement à un placebo une activité de l'acide pyridine-2,4-dicarboxylique et de son sel double de Na en faveur d'un accroissement ou d'un maintien de la densité des cheveux.

## Revendications

1. Utilisation d'une quantité efficace d'au moins un dérivé d'acide pyridine-dicarboxylique de formule générale (I) ou l'un de ses sels 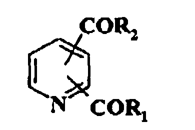 dans laquelle R₁ et R₂ représentent indépendamment l'un de l'autre OH, OR', -NH₂, -NHR', -NR'R", et
R' et R" représentent indépendamment l'un de l'autre, un radical alkyle en C₁-C₁₈, linéaire ou ramifié, saturé ou insaturé, ou un radical aryle, ce radical alkyle ou aryle étant éventuellement substitué par au moins un groupe OH, alkoxy, acyloxy, amino, ou alkylamino, ou R' et R" représentent ensemble un hétérocycle,
dans une composition cosmétique de soin et/ou de maquillage des fibres kératiniques humaines, pour induire et/ou stimuler la croissance des fibres kératiniques humaines et/ou freiner leur chute et/ou augmenter leur densité.

2. Utilisation d'une quantité efficace d'au moins un dérivé d'acide pyridine-dicarboxylique de formule générale (I) ou l'un de ses sels 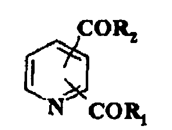 dans laquelle R₁ et R₂ représentent indépendamment l'un de l'autre OH, OR', -NH₂, -NHR', -NR'R", et
R' et R" représentent indépendamment l'un de l'autre, un radical alkyle en C₁-C₁₈, linéaire ou ramifié, saturé ou insaturé, ou un radical aryle, ce radical alkyle ou aryle étant éventuellement substitué par au moins un groupe OH, alkoxy, acyloxy, amino, ou alkylamino, ou R' et R" représentent ensemble un hétérocycle,
pour la préparation d'une composition de soin et/ou de traitement des fibres kératiniques humaines, destinée à induire et/ou stimuler la croissance des fibres kératiniques humaines et/ou freiner leur chute et/ou augmenter leur densité.

3. Utilisation cosmétique d'au moins un dérivé d'acide pyridine-dicarboxylique de formule générale (I) ou l'un de ses sels 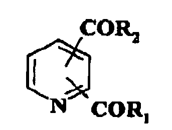 dans laquelle R₁ et R₂ représentent indépendamment l'un de l'autre OH, OR', -NH₂, -NHR', -NR'R", et
R' et R" représentent indépendamment l'un de l'autre, un radical alkyle en C₁-C₁₈, linéaire ou ramifié, saturé ou insaturé, ou un radical aryle, ce radical alkyle ou aryle étant éventuellement substitué par au moins un groupe OH, alkoxy, acyloxy, amino, ou alkylamino, ou R' et R" représentent ensemble un hétérocycle,
dans une composition cosmétique, comme agent pour induire et/ou stimuler la croissance des fibres kératiniques humaines et/ou freiner leur chute et/ou augmenter leur densité.

4. Utilisation selon l'une des revendications 1 à 3, **caractérisée en ce que** les fibres kératiniques sont les cheveux, les sourcils, les cils, les poils de barbe, de moustache, et les poils pubiens.

5. Dérivé d'acide pyridine-dicarboxylique de formule générale (I) ou l'un de ses sels 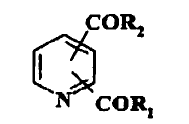 dans laquelle R₁ et R₂ représentent indépendamment l'un de l'autre OH. OR', -NH₂, -NHR', -NR'R", et
R' et R" représentent indépendamment l'un de l'autre, un radical alkyle en C₁-C₁₈, linéaire ou ramifié, saturé ou Insaturé, ou un radical aryle, ce radical alkyle ou aryle étant éventuellement substitué par au moins un groupe OH, alkoxy, acyloxy, amino, ou alkylamino, ou R' et R" représentent ensemble un hétérocycle,
pour utilisation dans un médicament pour traiter l'alopécie androgénique.

6. Utilisation selon l'une quelconque des revendications 1 à 4 dans laquelle fibres kératiniques humaines sont les cils.

7. Utilisation selon l'une des revendications précédentes, **caractérisée en ce que** R₁ et R₂ représentent indépendamment l'un de l'autre :
OH, OCH₃, OCH₂ - CH₃, 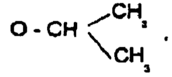 O-CH₂-O-COCH₃ , NHCH₂-CH₂ -CH₃ , NH-CH₂-CH₂OH, NH-CH₂-CH₂-CH₂OH, NH-CH₂-CH₂OCH₃.

8. Utilisation selon l'une des revendications précédentes, **caractérisée en ce que** R₁ et R₂ sont respectivement en position 2 et 3, ou 2 et 4 du noyau pyridine.

9. Utilisation selon l'une des revendications précédentes, **caractérisée en que** le sel du dérivé de formule (I) est un sel choisi parmi le sel double de sodium ou de potassium, les sels de zinc (Zn²⁺), de calcium (Ca²⁺), de cuivre (Cu²⁺), de fer (Fe²⁺), de strontium (Sr²⁺), de magnésium (Mg²⁺), de manganèse (Mn²⁺) les sels tri-éthanolamine, mono-éthanolamine, diéthanolamine, hexadécylamine, N,N,N',N'-tétrakis-(hydroxy-propyl-2) éthylène di-amine, tris-hydroxyméthylaminométhane, les hydroxydes, les carbonates, et les chlorures.

10. Utilisation selon l'une des revendications précédentes, **caractérisée en que** le dérivé d'acide pyridine-dicarboxylique est choisi parmi :
- l'acide pyridine-2,4-dicarboxylique ou son sel de zinc ou de sodium,
- l'acide pyridine-2,3-dicarboxylique ou son sel de zinc ou de sodium,
- le pyridine-2,4-dicarboxylate de diméthyle,
- le pyridine-2,3-dicarboxylate de diméthyle,
- le pyridine-2,4-dicarboxylate de diéthyle,
- le pyridine-2,3-dicarboxylate de diéthyle,
- le pyridine-2,4-dicarboxylate de di-isopropyle,
- la di(n-propylamido)-2,4-pyridine,
- le pyridine-2,4-dicarboxylate de di(acétyloxyméthyle),
- le pyridine-2,5-dicarboxylate de diéthyle,
- le pyridine-2,5-dicarboxylate de diméthyle,
- la di(2-hydroxy éthylamido)-2,4 pyridine,
- la di(3-hydroxy propylamido)-2,4 pyridine.

11. Utilisation selon l'une des revendications précédentes, **caractérisée en ce que** le dérivé est sous forme d'ester.

12. Utilisation selon l'une des revendications précédentes, **caractérisée en ce que** le dérivé est le pyridine-2,4 dicarboxylate de diéthyle.

13. Utilisation selon l'une des revendications précédentes, **caractérisée en que** le dérivé de formule (1) est utilisé en une quantité représentant de le % à 10%, de préférence de 10⁻² % à 5%, par rapport au poids total de la composition.

14. Utilisation selon l'une des revendications précédentes, **caractérisée en ce que** la composition est une composition à application topique.

15. Utilisation selon une des revendications précédentes, **caractérisée en ce que** la composition se présente sous forme de crème ou de lotion capillaire, de shampooing ou d'après-shampooing capillaire, de mascara pour les cils ou les cheveux.

16. Utilisation selon l'une des revendications précédentes, **caractérisée en ce que** la composition est sous forme de solution ou suspension aqueuse, alcoolique ou hydro-alcoolique.

17. Utilisation selon l'une des revendications précédentes, **caractérisée en ce que** la composition contient des adjuvants choisis parmi les gélifiants ou épaississants hydrophiles ou lipophiles, les additifs hydrophiles ou lipophiles, les conservateurs, les antioxydants, les solvants, les parfums, les charges, les absorbeurs d'odeurs, les électrolytes, les neutralisants, les agents bloqueurs d'U.V. comme les filtres solaires, les polymères filmogènes, les actifs cosmétiques et pharmaceutiques à action bénéfique pour la peau ou les fibres kératiniques, les matières colorantes, solubles ou non dans le milieu, les antagonistes de substance P, de CGRP ou de bradykinine et les inhibiteurs de NO synthase.

18. Utilisation selon l'une des revendications précédentes, **caractérisée en ce que** la composition comprend un composé actif additionnel favorisant la repousse des fibres kératiniques humaines et/ou limitant leur chute.

19. Utilisation selon la revendication 18, **caractérisée en ce que** le composé actif additionnel est choisi parmi :
- les hormones ;
- les agents antibactériens;
- les agents antagonistes de calcium ;
- les extraits de micro-organismes ;
- les agents modulant la différenciation et/ou la prolifération cutanée ;
- les agents modulant l'adhésion bactérienne sur la peau et /ou les muqueuses ;
- les antiparasitaires ;
- les antifongiques ;
- les agents antiviraux ;
- les agents anti-inflammatoires stéroïdiens ou non stéroïdiens ;
- les agents anesthésiques ;
- les agents antiprurigineux ;
- les agents kératolytiques ;
- les agents anti-radicaux libres ;
- les anti-séborrhéiques ;
- les antipelliculaires ;
- les antiacnéiques ;
- les vasodilatateurs ;
- les agents diminuant la chute des cheveux ;
- les agents antiandrogènes ;
- les inhibiteurs stéroïdiens ou non stéroïdiens des 5-α-réductases ;
- les agonistes potassiques ;
- les agonistes du récepteur FP ;
- leurs mélanges.

20. Utilisation selon la revendication 19, **caractérisée en ce que** l'actif favorisant la repousse des fibres kératiniques humaines et/ou limitant la chute est choisi parmi l'aminexil, le minoxidil, le latanoprost et le travoprost.

21. Composition de soin ou de maquillage des fibres kératiniques, comprenant dans un milieu physiologiquement acceptable au moins un dérivé de formule (I) ou l'une de ses sels, conforme à l'une des revendications 1 à 12, et au moins un actif additionnel favorisant la repousse des fibres kératiniques humaines et/ou limitant la chute choisi parmi l'aminexil, les agonistes du récepteur FP et les vasodilatateurs.

22. Composition selon la revendication 21, **caractérisée en ce que** l'actif additionnel est choisi parmi l'aminexil, le minoxidil, le latanoprost et le travoprost.

23. Composition selon la revendication 21 ou 22, **caractérisée en ce qu'**elle est à application topique.

24. Composition selon l'une des revendications 21 à 23, **caractérisée en ce qu'**elle se présente sous forme de crème ou lotion capillaire, de shampooing, d'après-shampoing capillaire, de mascara capillaire ou pour cils.

## Claims

1. Use of an effective amount of at least one pyridinedicarboxylic acid derivative of general formula (I) or one of its salts 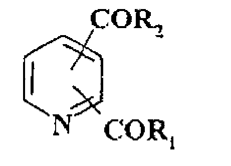 in which R₁ and R₂ represent, independently of one another, OH, OR', -NH₂, -NHR' or -NR'R", and
R' and R" represent, independently of one another, a saturated or unsaturated and linear or branched C₁-C₁₈ alkyl radical or an aryl radical, this alkyl or aryl radical optionally being substituted by at least one OH, alkoxy, acyloxy, amino or alkylamino group, or R' and R" together represent a heterocycle,
in a cosmetic composition for caring for and/or making up human keratinous fibres, to induce and/or stimulate the growth of human keratinous fibres and/or slow down their loss and/or increase their density.

2. Use of an effective amount of at least one pyridinedicarboxylic acid derivative of general formula (I) or one of its salts 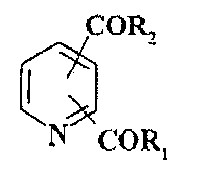 in which R₁ and R₂ represent, independently of one another, OH, OR', -NH₂, -NHR' or -NR'R", and
R' and R" represent, independently of one another, a saturated or unsaturated and linear or branched C₁-C₁₈ alkyl radical or an aryl radical, this alkyl or aryl radical optionally being substituted by at least one OH, alkoxy, acyloxy, amino or alkylamino group, or R' and R" together represent a heterocycle,
for the preparation of a composition for caring for or treating human keratinous fibres, intended to induce and/or stimulate the growth of human keratinous fibres and/or slow down their loss and/or increase their density.

3. Cosmetic use of at least one pyridinedicarboxylic acid derivative of general formula (I) or one of its salts 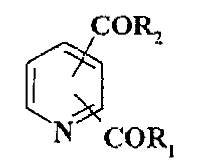 in which R₁ and R₂ represent, independently of one another, OH, OR', -NH₂, -NHR' or -NR'R", and
R' and R" represent, independently of one another, a saturated or unsaturated and linear or branched C₁-C₁₈ alkyl radical or an aryl radical, this alkyl or aryl radical optionally being substituted by at least one OH, alkoxy, acyloxy, amino or alkylamino group, or R' and R" together represent a heterocycle,
in a cosmetic composition, as agent for inducing and/or stimulating the growth of human keratinous fibres and/or slowing down their loss and/or increasing their density.

4. Use according to one of Claims 1 to 3, **characterized in that** the keratinous fibres are the hair, eyebrows, eyelashes, beard hairs, moustache hairs and pubic hairs.

5. Pyridinedicarboxylic acid derivative of general formula (I) or one of its salts 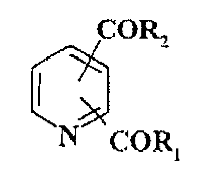 in which R₁ and R₂ represent, independently of one another, OH, OR', -NH₂, -NHR' or -NR'R", and
R' and R" represent, independently of one another, a saturated or unsaturated and linear or branched C₁-C₁₈ alkyl radical or an aryl radical, this alkyl or aryl radical optionally being substituted by at least one OH, alkoxy, acyloxy, amino or alkylamino group, or R' and R" together represent a heterocycle,
for use in a medicament for treating androgenic alopecia.

6. Use according to any one of Claims 1 to 4, in which the human keratinous fibres are the eyelashes.

7. Use according to one of the preceding claims, **characterized in that** R₁ and R₂ represent, independently of one another:
OH, OCH₃, OCH₂ - CH₃, 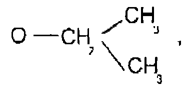 O-CH₂-O-COCH₃, NHCH₂-CH₂-CH₃, NH-CH₂-CH₂OH, NH-CH₂-CH₂-CH₂OH, NH-CH₂-CH₂OCH₃,

8. Use according to one of the preceding claims, **characterized in that** R₁ and R₂ are respectively in the 2 and 3 or 2 and 4 positions of the pyridine ring.

9. Use according to one of the preceding claims, **characterized in that** the salt of the derivative of formula (I) is a salt chosen from the double salt of sodium or of potassium, zinc (Zn²⁺), calcium (Ca²⁺) , copper (Cu²⁺) , iron (Fe²⁺) , strontium (Sr²⁺) , magnesium (Mg²⁺) or manganese (Mn²⁺) salts, triethanolamine, monoethanolamine, diethanolamine, hexadecylamine, N,N,N',N'-tetrakis(2-hydroxypropyl)ethylenediamine or tris(hydroxymethyl)aminomethane salts, hydroxides, carbonates or chlorides.

10. Use according to one of the preceding claims, **characterized in that** the pyridinedicarboxylate derivative is chosen from:
- pyridine-2,4-dicarboxylic acid or its zinc or sodium salt,
- pyridine-2,3-dicarboxylic acid or its zinc or sodium salt,
- dimethyl pyridine-2,4-dicarboxylate,
- dimethyl pyridine-2,3-dicarboxylate,
- diethyl pyridine-2,4-dicarboxylate,
- diethyl pyridine-2,3-dicarboxylate,
- diisopropyl pyridine-2,4-dicarboxylate,
- 2,4-di(n-propylamido)pyridine,
- di(acetyloxymethyl) pyridine-2,4-dicarboxylate,
- diethyl pyridine-2,5-dicarboxylate,
- dimethyl pyridine-2,5-dicarboxylate,
- 2,4-di(2-hydroxyethylamido)pyridine,
- 2,4-di(3-hydroxypropylamido)pyridine.

11. Use according to one of the preceding claims, **characterized in that** the derivative is in the ester form.

12. Use according to one of the preceding claims, **characterized in that** the derivative is diethyl pyridine-2,4-dicarboxylate.

13. Use according to one of the preceding claims, **characterized in that** the derivative of formula (I) is used in an amount representing from 10⁻³% to 10%, preferably from 10⁻²% to 5%, with respect to the total weight of the composition.

14. Use according to one of the preceding claims, **characterized in that** the composition is a composition for topical application.

15. Use according to one of the preceding claims, **characterized in that** the composition is provided in the form of a hair cream or lotion, of a shampoo or hair conditioner, or of a mascara for the eyelashes or hair.

16. Use according to one of the preceding claims, **characterized in that** the composition is in the form of an aqueous, alcoholic or aqueous/alcoholic solution or suspension.

17. Use according to one of the preceding claims, **characterized in that** the composition comprises adjuvants chosen from hydrophilic or lipophilic gelling agents or thickeners, hydrophilic or lipophilic additives, preservatives, antioxidants, solvents, fragrances, fillers, odour absorbers, electrolytes, neutralizing agents, UV blocking agents, such as sunscreens, film-forming polymers, cosmetic and pharmaceutical active principles with a beneficial effect on the skin or keratinous fibres, colouring materials, which may or may not be soluble in the medium, substance P, CGRP or bradykinin antagonists or NO-synthase inhibitors.

18. Use according to one of the preceding claims, **characterized in that** the composition comprises an additional active compound which promotes the regrowth of human keratinous fibres and/or which limits their loss.

19. Use according to Claim 18, **characterized in that** the additional active compound is chosen from:
- hormones;
- antibacterial agents;
- calcium antagonist agents;
- microorganism extracts;
- agents which modulate cutaneous differentiation and/or proliferation;
- agents which modulate bacterial adhesion to the skin and/or mucous membranes;
- agents for combating parasites;
- antifungals;
- antiviral agents;
- steroidal or nonsteroidal anti-inflammatory agents;
- anaesthetic agents;
- antipruriginous agents;
- keratolytic agents;
- agents for combating free radicals;
- antiseborrheics;
- antidandruff agents;
- anti-acne agents;
- vasodilators;
- agents which reduce hair loss;
- anti-androgen agents;
- steroidal or nonsteroidal inhibitors of 5α-reductases;
- potassium channel agonists;
- FP receptor agonists;
- their mixtures.

20. Use according to Claim 19, **characterized in that** the active principle which promotes the regrowth of human keratinous fibres and/or which limits the loss is chosen from aminexil, minoxidil, latanoprost and travoprost.

21. Composition for caring for or making up keratinous fibres comprising, in a physiologically acceptable medium, at least one derivative of formula (I) or one of its salts, in accordance with one of Claims 1 to 12, and at least one additional active principle which promotes the regrowth of human keratinous fibres and/or which limits the loss chosen from aminexil, FP receptor agonists and vasodilators.

22. Composition according to Claim 21, **characterized in that** the additional active principle is chosen from aminexil, minoxidil, latanoprost and travoprost.

23. Composition according to Claim 21 or 22, **characterized in that** it is for topical application.

24. Composition according to one of Claims 21 to 23, **characterized in that** it is provided in the form of a hair cream or lotion, of a shampoo, of a hair conditioner, or of a hair mascara for the eyelashes.

## Patentansprüche

1. Verwendung einer wirksamen Menge mindestens eines Pyridindicarbonsäurederivates der allgemeinen Formel (I) oder eines seiner Salze: 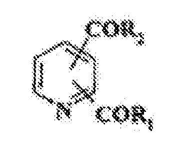 in der Formel:
R₁ und R₂ bedeuten unabhängig voneinander OH, OR', -NH₂, -NHR', -NR'R", und□
R' und R" bedeuten unabhängig voneinander eine geradkettige oder verzweigte, gesättigte oder ungesättigte C₁₋₁₈-Alkylgruppe oder eine Arylgruppe, wobei die Alkyl- oder Arylgruppe gegebenenfalls mit mindestens einer Gruppe OH, Alkoxy, Acyloxy, Amino oder Alkylamino substituiert ist, oder R' und R" bedeuten gemeinsam einen Heterocyclus. □ in einer kosmetischen Zusammensetzung für die Pflege und/oder zum Schminken von menschlichen Keratinfasern, um das Wachstum von menschlichen Keratinfasern zu induzieren und/ oder zu stimulieren und/ oder ihr Ausfallen zu verlangsamen und/oder ihre Dichte zu vergrößern.

2. Verwendung einer wirksamen Menge mindestens eines Pyridindicarbonsäurederivates der allgemeinen Formel (I) oder eines seiner Salze: 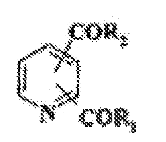 in der Formel:
R₁ und R₂ bedeuten unabhängig voneinander OH, OR', -NH₂, -NHR', -NR'R", und□
R' und R" bedeuten unabhängig voneinander eine geradkettige oder verzweigte, gesättigte oder ungesättigte C₁₋₁₈-Alkylgruppe oder eine Arylgruppe, wobei die Alkyl- oder Arylgruppe gegebenenfalls mit mindestens einer Gruppe OH, Alkoxy, Acyloxy, Amino oder Alkylamino substituiert ist, oder R' und R" bedeuten gemeinsam einen Heterocyclus, □ zur Herstellung einer Zusammensetzung für die Pflege und/oder zur Behandlung von menschlichen Keratinfasern, die dazu vorgesehen ist, das Wachstum von menschlichen Keratinfasern zu induzieren und/oder zu stimulieren und/oder ihr Ausfallen zu verlangsamen und/oder ihre Dichte zu vergrößern.

3. Kosmetische Verwendung mindestens eines Pyridindicarbonsäurederivates der allgemeinen Formel (I) oder eines seiner Salze: 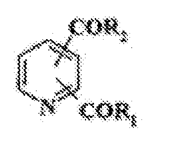 in der Formel:
R₁ und R₂ bedeuten unabhängig voneinander OH, OR', -NH₂, -NHR', -NR'R", und□
R' und R" bedeuten unabhängig voneinander eine geradkettige oder verzweigte, gesättigte oder ungesättigte C₁₋₁₈-Alkylgruppe oder eine Arylgruppe, wobei die Alkyl- oder Arylgruppe gegebenenfalls mit mindestens einer Gruppe OH, Alkoxy, Acyloxy, Amino oder Alkylamino substituiert ist, oder R' und R" bedeuten gemeinsam einen Heterocyclus,□ in einer kosmetischen Zusammensetzung als Mittel, um das Wachstum von menschlichen Keratinfasern zu induzieren und/ oder zu stimulieren und/ oder ihr Ausfallen zu verlangsamen und/oder ihre Dichte zu vergrößern.

4. Verwendung nach einem der Ansprüche 1 bis 3, **dadurch gekennzeichnet, dass** es sich bei den Keratinfasern um Haare, Augenbrauen, Wimpern, Barthaare, Schnurrbarthaare oder Schamhaare handelt.

5. Pyridindicarbonsäurederivat der allgemeinen Formel (I) oder eines seiner Salze: 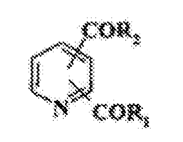 in der Formel:
R₁ und R₂ bedeuten unabhängig voneinander OH, OR', -NH₂, -NHR', -NR'R", und□
R' und R" bedeuten unabhängig voneinander eine geradkettige oder verzweigte, gesättigte oder ungesättigte C₁₋₁₈-Alkylgruppe oder eine Arylgruppe, wobei die Alkyl- oder Arylgruppe gegebenenfalls mit mindestens einer Gruppe OH, Alkoxy, Acyloxy, Amino oder Alkylamino substituiert ist, oder R' und R" bedeuten gemeinsam einen Heterocyclus,□
zur Verwendung in einem Arzneimittel zur Behandlung androgener Alopezie.

6. Verwendung nach einem der Ansprüche 1 bis 4, wobei es sich bei den menschlichen Keratinfasern um Wimpern handelt.

7. Verwendung nach einem der vorhergehenden Ansprüche, **dadurch gekennzeichnet, dass** R₁ und R₂ unabhängig voneinander bedeuten:
OH, OCH₂, OCH₃-CH₃, 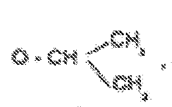 O-CH₃-O-COOH₂, NHCH₂ -CH₂-CH₃ NH-CH₂CH₂OH_{,} NH-CH₂-CH₃-CH₂-OH, NH-CH₂-CH₃-OCH₃.

8. Verwendung nach einem der vorhergehenden Ansprüche,
**dadurch gekennzeichnet, dass** sich die Gruppen R₁ und R₂ in 2- bzw. 3-Stellung oder 2- bzw. 4-Stellung des Pyridinrings befinden.

9. Verwendung nach einem der vorhergehenden Ansprüche,
**dadurch gekennzeichnet, dass** das Salz des Derivates der Formel (I) ein Salz ist, das unter den Doppelsalzen von Natrium oder Kalium, den Salzen von Zink (Zn²⁺), Calcium (Ca²⁺), Kupfer (Cu²⁺), Eisen (Fe²⁺), Strontium (Sr²⁺), Magnesium (Mg²⁺), Mangan (Mn²⁺), Triethanolaminsalzen, Monoethanolaminsalzen, Diethanolaminsalzen, Hexadecylaminsalzen, N,N,N',N'-Tetrakis-(2-hydroxy-propyl)ethylendiaminsalzen, Trishydroxymethylaminomethansalzen, Hydroxiden, Carbonaten und Chloriden ausgewählt ist.

10. Verwendung nach einem der vorhergehenden Ansprüche,
**dadurch gekennzeichnet, dass** das Pyridindicarbonsäurederivat ausgewählt ist unter:
- Pyridin-2,4-dicarbonsäure oder ihrem Zinksalz oder Natriumsalz,
- Pyridin-2,3-dicarbonsäure oder ihrem Zinksalz oder Natriumsalz,
- Dimethyl-pyridin-2,4-dicarboxylat,
- Dimethyl-pyridin-2,3-dicarboxylat,
- Diethyl-pyridin-2,4-dicarboxylat,
- Diethyl-pyridin-2,3-dicarboxylat,
- Diisopropyl-pyridin-2,4-dicarboxylat,
- Di(n-propylamido)-2,4-pyridin,
- Di(acetyloxymethyl)-pyridin-2,4-dicarboxylat,□
- Diethyl-pyridin-2,5-dicarboxylat,
- Dimethyl-pyridin-2, 5-dicarboxylat,
- Di(2-hydroxy-ethylamido)-2,4-pyridin, □
- Di(3-hydroxy-propylamido)-2,4-pyridin. □ □ □

11. Verwendung nach einem der vorhergehenden Ansprüche,
**dadurch gekennzeichnet, dass** das Derivat als Ester vorliegt.

12. Verwendung nach einem der vorhergehenden Ansprüche,
**dadurch gekennzeichnet, dass** es sich bei dem Derivat um das Diethyl-pyridin-2,4-dicarboxylat handelt.

13. Verwendung nach einem der vorhergehenden Ansprüche,
**dadurch gekennzeichnet, dass** das Derivat der Formel (I) in einem Mengenanteil von 10⁻³ bis 10 % und vorzugsweise 10⁻² bis 5 %, bezogen auf das Gesamtgewicht der Zusammensetzung, verwendet wird.

14. Verwendung nach einem der vorhergehenden Ansprüche,
**dadurch gekennzeichnet, dass** die Zusammensetzung eine Zusammensetzung zur topischen Anwendung ist.

15. Verwendung nach einem der vorhergehenden Ansprüche,
**dadurch gekennzeichnet, dass** die Zusammensetzung in Form einer Creme oder Lotion für die Haarbehandlung, als Haarwaschmittel oder haarkosmetisches Produkt, das nach der Haarwäsche angewandt wird, Mascara für die Wimpern oder die Haare vorliegt.

16. Verwendung nach einem der vorhergehenden Ansprüche,
**dadurch gekennzeichnet, dass** die Zusammensetzung als wässrige, alkoholische oder wässrig-alkoholische Suspension oder Lösung vorliegt.

17. Verwendung nach einem der vorhergehenden Ansprüche,
**dadurch gekennzeichnet, dass** die Zusammensetzung Zusatzstoffe enthält, die ausgewählt sind unter: hydrophilen oder lipophilen Gelbildnern oder Verdickungsmitteln, hydrophilen oder lipophilen Additiven, Konservierungsmitteln, Antioxidantien, Lösemitteln, Parfums, Füllstoffen, Geruchsabsorbern, Elektrolyten, Neutralisationsmitteln, UV-Blockern, wie Sonnenschutzfiltern, filmbildenden Polymeren, kosmetischen oder pharmazeutischen Wirkstoffen mit vorteilhafter Wirkung auf Haut oder Keratinfasern, Farbmitteln, die in dem Medium löslich sind oder nicht, Substanz-P-Antagonisten, CGRP-Antagonisten oder Bradykinin-Antagonisten und Inhibitoren der NO-Synthase.

18. Verwendung nach einem der vorhergehenden Ansprüche,
**dadurch gekennzeichnet, dass** die Zusammensetzung einen ergänzenden Wirkstoff enthält, der das Wachstum von menschlichen Keratinfasern fördert und/ oder ihr Ausfallen einschränkt.

19. Verwendung nach Anspruch 18, **dadurch gekennzeichnet, dass** der ergänzende Wirkstoff ausgewählt ist unter:
· Hormonen;
· antibakteriellen Wirkstoffen;
· Calcium-Antagonisten;
· Extrakten von Mikroorganismen;
· Stoffen, die die Differenzierung und/ oder Proliferation der Haut verändern;
· Stoffen, die die bakterielle Haftung von Bakterien auf der Haut und/ oder den Schleimhäuten verändern;
· antiparasitären Wirkstoffen;
· antimykotischen Wirkstoffen;
· antiviralen Wirkstoffen;
· steroidalen oder nicht steroidalen entzündungshemmenden Wirkstoffen;
· anästhesierenden Wirkstoffen;
· juckreizstillenden Wirkstoffen;
· Keratolytika;
· Radikalfängern für freie Radikale;
· Antiseborrhoika;
· Antischuppenmitteln;
· Wirkstoffen gegen Akne;
· Vasodilatoren;
· Wirkstoffen, die das Ausfallen der Haare vermindern;
· Antiandrogenen;
· steroidalen oder nicht steroidalen Inhibitoren der 5-α-Reductasen;
· Kalium-Agonisten;
· Rezeptor FP-Agonisten;
· deren Gemischen.

20. Verwendung nach Anspruch 19, **dadurch gekennzeichnet, dass** der Wirkstoff, der das Wachstum von menschlichen Keratinfasern fördert und/ oder ihr Ausfallen einschränkt, unter Aminexil, Minoxidil, Latanoprost und Travoprost ausgewählt ist.

21. Zusammensetzung für die Pflege oder zum Schminken von Keratinfasern, die in einem physiologisch akzeptablen Medium mindestens ein Derivat der Formel (I) oder eines seiner Salze gemäß einem der Ansprüche 1 bis 12 und mindestens einen ergänzenden Wirkstoff enthält, der das Wachstum von menschlichen Keratinfasern fördert und/oder ihr Ausfallen einschränkt und der unter Aminexil, Rezeptor FP-Agonisten und Vasodilatoren ausgewählt ist.

22. Zusammensetzung nach Anspruch 21, **dadurch gekennzeichnet, dass** der ergänzende Wirkstoff unter Aminexil, Minoxidil, Latanoprost und Travoprost ausgewählt ist.

23. Zusammensetzung nach Anspruch 21 oder 22, **dadurch gekennzeichnet, dass** sie zur topischen Anwendung vorgesehen ist.

24. Zusammensetzung nach einem der Ansprüche 21 bis 23,
**dadurch gekennzeichnet, dass** sie in Form einer Creme oder Lotion für die Haarbehandlung, als Haarwaschmittel oder haarkosmetisches Produkt, das nach der Haarwäsche angewandt wird, Mascara für die Haare oder für die Wimpern vorliegt.
